# EUROPÄISCHE PATENTSCHRIFT

(11) **EP 0 927 040 B1**
(45) Veröffentlichungstag und Bekanntmachung des Hinweises auf die Patenterteilung: **14.04.2004**
(21) Anmeldenummer: 96930986.3
(22) Anmeldetag: 30.08.1996
(51) Int. Cl.: A61K 35/22, C07J 75/00

(54) **PROZESS ZUR GEWINNUNG VON OESTROGENEN AUS STUTENHARN**
PROCESS TO OBTAIN OESTROGENS FROM MARE'S URINE
PROCEDE D'OBTENTION D'OESTROGENES A PARTIR D'URINE DE JUMENT

(43) Veröffentlichungstag der Anmeldung: 07.07.1999
(73) Patentinhaber: Solvay Pharmaceuticals GmbH, 30173 Hannover (DE)
(72) Erfinder: BAN, Ivan, D-30539 Hannover (DE); BORCHERS, Friedrich, D-30880 Laatzen (DE); HEINEMANN, Henning, D-31275 Lehrte (DE); RASCHE, Heinz-Helmer, D-31303 Burgdorf (DE)
(74) Vertreter: Gosmann, Martin
(86) Internationale Anmeldenummer: PCT/EP1996/003819
(87) Internationale Veröffentlichungsnummer: WO 1998/008525

(56) Entgegenhaltungen:
- CLINICAL CHEMISTRY, Bd. 27, Nr. 7, 1981, Seiten 1186-1189, XP000673641 HEIKKINEN R. ET AL: "Reversed-phase C18 cartridge for extraction of estrogens from urine and plasma" in der Anmeldung erwähnt
- CLINICA CHIMICA ACTA, Bd. 107, 1980, Seiten 231-243, XP000673611 SHACKLETON C.H.L. ET AL: "Use of Sep-Pak cartridges for urinary steroid extraction: evaluation of the method for use prior to gas chromatography" in der Anmeldung erwähnt

## Beschreibung

Die vorliegende Erfindung betrifft die Gewinnung eines natürlichen Gemisches konjugierter Oestrogene aus dem Harn trächtiger Stuten.

Oestrogene werden in der Medizin zur Hormonsubstitutionstherapie eingesetzt. Insbesondere werden Oestrogengemische zur Behandlung und Prophylaxe der bei Frauen auftretenden Beschwerden der Wechseljahre nach natürlicher oder artifizieller Menopause eingesetzt. Hierbei haben sich natürliche Gemische konjugierter Oestrogene, wie sie im Harn trächtiger Stuten vorliegen, als besonders wirksam und gut verträglich erwiesen.

Der gelöste Feststoffgehalt im Harn trächtiger Stuten (=pregnant mares urine, im folgenden abgekürzt als "PMU") kann natürlicherweise in weiten Bereichen schwanken und im allgemeinen in einem Bereich von 40 - 90 g Trockensubstanz pro Liter liegen. Neben Harnstoff und sonstigen üblichen Harninhaltsstoffen sind in dem Feststoffgehalt des PMU phenolische Bestandteile in Mengen von ca. 2 - 5 Gew.% bezogen auf Trockensubstanz enthalten. Unter diesen phenolischen Bestandteilen befinden sich Kresole und das als HPMF bekannte Dihydro-3,4-bis[(3-hydroxyphenyl)methyl]-2(3H)-furanon. Diese können in freier oder konjugierter Form vorliegen. In dem PMU ist ein natürliches Gemisch von Oestrogenen enthalten, welches weitgehend in konjugierter Form, z.B. als Schwefelsäurehalbester-Natriumsalz (im folgenden abgekürzt als "Sulfatsalz"), vorliegt. Der Gehalt an konjugierten Oestrogenen (berechnet als Oestrogensulfatsalz) kann zwischen 0,3 und 1 Gew. % bezogen auf Trockensubstanz betragen.

Üblicherweise werden konjugierte Oestrogene enthaltende Extrakte aus dem PMU durch Extraktion mit einem polaren mit Wasser nicht oder nur wenig mischbaren organischen Lösemittel wie beispielsweise Essigsäureethylester, n-Butanol oder Cyclohexanol, gewonnen. Bei derartigen Flüssig-flüssig-Extraktionen treten jedoch eine Vielzahl von Problemen, wie starke Schaumbildung, Sedimentbildung, Emulsionsbildung und schlechte Phasentrennung auf. Es werden im allgemeinen mehrere Extraktionsschritte benötigt, was zu Verlusten und einer nur teilweisen Gewinnung des Oestrogengehaltes führt.

Für die Aufbereitung von kleinen Mengen an Harn- und Plasmaflüssigkeiten für eine analytische Bestimmung von Oestrogenen mittels Gaschromatographie wird von Heikkinnen et al. (Clin. Chem. 27/7,(1981), 1186 - 1189) und von Shackleton et al. (Clinica Chimica Acta 107(1980) 231-243) eine Festphasen-Extraktion von Oestrogenen mittels einer Kartusche mit Octadecylsilanreste enthaltendem silanisiertem Kieselgel (Sep-Pak^{R} C₁₈-cartridge, Hersteller Waters Ass. Inc.Milford, MA, USA) vorgeschlagen. Hierbei werden die Oestrogene mit Methanol von der Kartusche eluiert. Es werden jedoch keine Angaben über die weiteren in dem oestrogenhaltigen Eluat enthaltenen Stoffe gemacht.

Aufgabe der vorliegenden Erfindung ist es, ein technisches Verfahren zur Gewinnung des natürlichen Gemisches konjugierter Oestrogene aus dem PMU unter Vermeidung der von den bisher üblichen Flüssig-flüssig-Extraktionen bekannten Nachteile zu entwickeln, welches ein an phenolischen Harninhaltsstoffen abgereichertes weitgehend HPMF-freies Produkt liefert.

Es wurde nun ein Verfahren gefunden, mit welchem in einer Festphasen-Extraktion an hydrophobisiertem Kieselgel ein an phenolischen Harninhaltsstoffen abgereichertes, weitgehend HPMF-freies, jedoch den natürlichen Oestrogen-Gehalt des PMU praktisch vollständig enthaltendes Gemisch gewonnen werden kann, welches als Ausgangsmaterial für die Herstellung von das natürliche Gemisch konjugierter Oestrogene aus dem PMU als Wirkkomponente enthaltenden Pharmazeutika dienen kann.

Das erfindungsgemäße Verfahren zur Gewinnung eines an phenolischen Harninhaltsststoffen abgereicherten natürlichen Gemisches konjugierter Oestrogene aus dem PMU ist dadurch gekennzeichnet, daß man
a) eine Harnflüssigkeit, welche den von Schleim- und Feststoffen befreiten Harn, ein eingeengtes Konzentrat dieses Harns oder ein durch Ultrafiltration dieses Harns erhaltenes eingeengtes Harnretentat darstellt, mit einer zur Einstellung eines pH-Wertes von mindestens 12 ausreichenden Menge einer wasserlöslichen Base behandelt und anschließend den pH-Wert der mit der Base behandelten Harnflüssigkeit durch Zugabe einer ausreichenden Menge einer wäßrigen Säurelösung auf einen pH-Wert im Bereich von 5 bis 8,5 einstellt,
b) die mit der Base vorbehandelte, auf pH 5 bis 8,5 eingestellte Harnflüssigkeit mit einer zur Adsorption des in der Harnflüssigkeit enthaltenen Gemisches konjugierter Oestrogene ausreichenden Menge eines hydrophobisierten Kieselgels kontaktiert und ein mit dem Gemisch konjugierter Oestrogene beladenes hydrophobisiertes Kieselgel von der übrigen Harnflüssigkeit abtrennt,
c) das mit dem Gemisch konjugierter Oestrogene beladene hydrophobisierte Kieselgel mit einer wäßrigen auf einen pH-Bereich von 5 bis 7, insbesondere 5, eingestellten Pufferlösung wäscht,
d) das gewaschene hydrophobisierte Kieselgel mit einer zur Desorption des daran adsorbierten Gemisches konjugierter Oestrogene ausreichenden Menge einer Elutionsflüssigkeit, welche ein Gemisch aus Wasser und einem mit Wasser mischbaren organischen Lösemittel aus der Gruppe mit Wasser mischbarer Ether, niederer Alkanole und niederer aliphatischer Ketone darstellt, kontaktiert und ein das natürliche Gemisch konjugierter Oestrogene enthaltendes Eluat von dem hydrophobisierten Kieselgel abtrennt und gegebenenfalls einengt.

Für das erfindungsgemäße Verfahren können der PMU als solcher, ein daraus durch Einengen gewonnenes Konzentrat oder ein daraus durch Membranfiltration gewonnenes Retentat eingesetzt werden. Der gesammelte Harn wird zunächst auf an sich bekannte Weise von Schleim- und Feststoffen befreit. Zweckmäßig läßt man Fest- und Schleimstoffe absetzen und trennt diese dann nach an sich bekannten Trennungsmethoden, beispielsweise Dekantieren, Separieren und/oder Filtrieren ab. So kann der PMU beispielsweise über eine an sich bekannte Trenneinrichtung, z. B. einen Separator, eine Filtrationsanlage oder einen Sedimentator geleitet werden. Als Trenneinrichtung kann z. B. ein Sandbett dienen, oder es können handelsübliche Separatoren, z. B. Düsenoder Kammerseparatoren, eingesetzt werden. Gewünschtenfalls können auch eine Microfiltrationsanlage oder eine Ultrafiltrationsanlage eingesetzt werden, bei deren Verwendung gleichzeitig eine weitgehende Keim- und Virenfreiheit des filtrierten PMU erreicht werden kann.

Gewünschtenfalls können dem Harn Konservierungsmittel, Germizide, Bakterizide und/oder Anthelmintika zugesetzt werden.

Sofern anstelle des PMU ein aufkonzentriertes PMU-Retentat eingesetzt werden soll, kann dieses aus dem PMU durch an sich bekannte Membranfiltration gewonnen werden. Der Feststoffgehalt des Retentates und dessen Zusammensetzung können je nach dem eingesetzten PMU und der zur Membranfiltration verwendeten Membran, beispielsweise deren Porenweite, sowie den Bedingungen der Filtration variieren. Beispielsweise kann bei Verwendung einer Nanofiltrationsmembran eine nahezu verlustfreie Konzentration des Oestrogengehaltes in dem PMU-Retentat bei gleichzeitiger Entfernung von bis zu 50 Gew.-% der niedermolekularen PMU-Inhaltsstoffe erreicht werden. Für das erfindungsgemäße Verfahren können PMU-Retentate eingesetzt werden, welche bis zu einem Verhältnis von ca. 1 : 10, beispielsweise einem Verhältnis von ca. 1 : 7 aufkonzentriert wurden und deren Volumen somit bis auf ca. 1/10, beispielsweise ca. 1/7, des ursprünglichen PMU-Volumens eingeengt sein kann.

Im Verfahrenssschritt a) wird der Harnflüssigkeit eine zur Einstellung eines pH-Wertes von mindestens 12, insbesondere 13 bis 14, ausreichende Menge einer wasserlöslichen Base zugesetzt. Als wasserlösliche Basen eignen sich anorganische oder organische in der Harnflüssigkeit lösliche inerte Basen, welche stark genug sind, um einen pH-Wert von mindestens 12 zu erreichen. So eignen sich Alkalimetall- oder Erdalkalimetallhydroxide, insbesondere Natriumhydroxid, oder auch organische Basen wie quartäre Niederalkylammoniumhydroxide. Vorzugsweise wird die Base in Form einer wäßrigen Lösung mit einem Basengehalt von mindestens 20 Gew.-%, vorzugsweise 40 bis 60 Gew.-%, zugesetzt. Als zweckmäßig erweist sich insbesondere eine 45- bis 55-%ige wäßrige Natronlauge. Bei dieser alkalischen Vorbehandlung wird der pH-Wert der Harnflüssigkeit auf mindestens 12, beispielsweise einen Bereich von 12 bis 14, vorzugsweise ca. 13, eingestellt und dieser alkalische pH-Wert wird für eine zur Spaltung von in Harninhaltsstoffen enthaltenen Lactongruppierungen ausreichende Zeitdauer, welche im allgemeinen 1/2 bis 2 Stunden betragen kann, aufrechterhalten. Die Behandlungsbedingungen müssen so gewählt werden, daß die konjugierten Oestrogene dabei nicht angegriffen werden. So findet die Behandlung bevorzugt bei Raumtemperatur unter Beschränkung der Basenzugabe auf die zum Erreichen des gewünschten pH-Wertes im Bereich von 12 bis 14 benötigte Menge statt. Die zum Erreichen eines pH-Wertes im Bereich von 12 bis 14 benötigte Menge Base kann je nach Zusammensetzung des eingesetzten PMU variieren. Bei Verwendung von 50-%iger Natronlauge erweisen sich im allgemeinen Mengen von ca. 100 bis 700 ml 50-%iger Natronlauge bezogen auf 10 1 PMU als ausreichend. Anschließend wird die Harnflüssigkeit durch Zugabe einer wäßrigen Säurelösung auf einen pH-Wert im Bereich von 5 bis 8,5, vorzugsweise 7 bis 8,5, insbesondere 8 bis 8,5 eingestellt. Als Säuren eignen sich in der Harnflüssigkeit lösliche inerte anorganische oder organische Säuren wie beispielsweise Salzsäure, Schwefelsäure, Phosphorsäure oder niedere Carbonsäuren wie Essigsäure. Als zweckmäßig erweist sich beispielsweise eine konzentrierte wäßrige Salzsäurelösung. Die zum Erreichen des vorgenannten pH-Bereiches notwendige Säuremenge kann je nach Zusammensetzung des eingesetzten PMU und der zur Alkalischstellung verwendeten Basenmenge variieren. Bei Verwendung von konzentrierter Salzsäurelösung kann der gewünschte pH-Wert im allgemeinen durch Zugabe von ca. 25 bis 100 g konzentrierter Salzsäurelösung bezogen auf 1 1 PMU erzielt werden.

Die in dem Verfahrensschritt b) einsetzbaren hydrophobisierten Kieselgele sind an sich bekannte Umkehr-Phasen-Kieselgele (=Reverse-Phase-Kieselgele, abgekürzt "RP-Kieselgele"), das sind chemisch modifizierte Kieselgele, die hydrophobe funktionelle Gruppen bzw. Liganden tragen. So eignen sich beispielsweise silanisierte RP-Kieselgele, welche als hydrophobe funktionelle Gruppen n-Octadecyldimethylsilyloxy-, n-Octyldimethylsilyloxy- oder Dimethylhydroxysilyloxy-Reste enthalten. Geeignet sind z. B. silanisierte Kieselgele mit mittleren Korngrößen von 15 bis 500 µm. Als besonders zweckmäßig hat sich Dimethylhydroxylsilyloxy-Reste enthaltenes Kieselgel mit einer mittleren Korngröße im Bereich von 0,05 - 0,3 mm, beispielsweise das "Kieselgel 60/Dimethylsilanderivat" der Firma Merck, erwiesen.

Erfindungsgemäß kann die Adsorption der konjugierten Oestrogene an das hydrophobisierte Kieselgel durch Kontaktierung des gegebenenfalls aufkonzentrierten PMU oder dessen Retentats mit dem hydrophobisierten Kieselgel erfolgen, indem die in dem Verfahrensschritt a) vorbehandelte Harnflüssigkeit in einen das Kieselgel enthaltenden Reaktor eingeführt und darin für eine zur Adsorption des Oestrogengehaltes ausreichende Zeit mit dem Kieselgel in Kontakt gehalten wird. Nach erfolgter Adsorption der konjugierten Oestrogene an das hydrophobisierte Kieselgel kann das mit dem Gemisch konjugierter Oestrogene beladene Kieselgel von der übrigen Harnflüssigkeit auf an sich bekannte Weise abgetrennt werden. Zweckmäßigerweise kann die Harnflüssikgeit durch eine das Kieselgel enthaltende Säule mit einer solchen Durchlaufgeschwindigkeit geleitet werden, daß die Kontaktzeit zur Adsorption des Oestrogengehaltes ausreicht. Geeignet sind beispielsweise Durchlaufgeschwindigkeiten, welche einem Durchlauf von 5 bis 20 Vol.-Teilen PMU/1 Vol.-Teil Kieselgel/Stunde entsprechen. Die Adsorption wird bevorzugt bei Raumtemperatur durchgeführt. Zweckmäßig kann die Durchlaufgeschwindigkeit der Harnflüssigkeit durch den Reaktor durch Arbeiten bei geringem Überdruck oder Unterdruck gesteuert werden. Die Menge an anzuwendendem hydrophobisiertem Kieselgel kann je nach Art des verwendeten Kieselgels und Menge des Feststoff-Gehaltes in der im Verfahrensschritt a) vorbehandelten Harnflüssigkeit variieren. Bei Verwendung von vorbehandeltem PMU kann beispielsweise ein Volumenteil hydrophobisiertes Kieselgel mit bis zu 80 Volumen-Teilen vorbehandeltem PMU beladen werden, ohne daß merkliche Mengen Oestrogen in der abfließenden Harnflüssigkgeit nachweisbar sind. Bei Verwendung eines vorbehandelten PMU-Konzentrates oder PMU-Retentates reduziert sich die Beladungskapazität des hydrophobisierten Kieselgels natürlich in dem Maße, in dem diese aufkonzentriert sind. So kann beispielsweise 1 Volumenteil an hydrophobisiertem Kieselgel mit einer 30 bis 80, vorzugsweise 40 bis 50, Volumenteilen PMU entsprechenden Menge Harnflüssigkeit beladen werden.

Das mit dem Gemisch konjugierter Oestrogene beladene hydrophobisierte Kieselgel wird in Verfahrensschritt c) mit einer wäßrigen auf einen pH-Bereich von 5 bis 7, vorzugsweise ca. 5, eingestellten Pufferlösung gewaschen. Als Waschflüssigkeit wird vorzugsweise eine auf ca. pH 5 eingestellte wäßrige Essigsäure/Acetat-Pufferlösung eingesetzt. Es eignen sich insbesondere 0,1 bis 1 molare, bevorzugt 0,1 bis 0,2-molare, Essigsäure/Acetat-Pufferlösungen. Die Menge an Waschflüssigkeit wird so gewählt, daß sie ausreicht, um phenolische Harninhaltsstoffe weitgehend auszuwaschen, ohne daß dabei nennenswerte Mengen konjugierter Oestrogene mit ausgewaschen werden. Als zweckmäßig erweist sich beispielsweise die Verwendung von 8 bis 15, insbesondere 9 bis 10, Bettvolumen Waschflüssigkeit pro Bettvolumen hydrophobisiertes Kieselgel. Hierbei wird die Waschflüssigkeit zweckmäßigerweise durch einen das hydrophobisierte Kieselgel enthaltenden Reaktor mit einer Durchflußgeschwindigkeit von 5 bis 20 Vol.-Teilen Waschflüssigkeit /1 Vol.-Teil Kieselgel/-Stunde geleitet.

Im Verfahrensschritt d) wird anschließend das gewaschene mit dem Gemisch konjugierter Oestrogene beladene hydrophobisierte Kieselgel mit einer zur Elution des Gemisches der konjugierten Oestrogene ausreichenden Menge einer Elutionsflüssigkeit behandelt und ein das natürliche Gemisch der konjugierten Oestrogene des PMU enthaltendes Eluat gewonnen. Die erfindungsgemäß verwendete Elutionsflüssigkeit stellt ein Gemisch aus Wasser und einem mit Wasser mischbaren Ether, niederen Alkanol und/oder niederen aliphatischen Keton dar. Als Etherbestandteile der Elutionsflüssigkeit eignen sich mit Wasser mischbare cyclische Ether wie Tetrahydrofuran oder Dioxan aber auch mit Wasser mischbare offenkettige Ether wie beispielsweise Ethylenglycoldimethylether (=Monoglyme), Diethylenglycoldimethylether (=Diglyme) oder Ethyloxyethyloxyethanol (=Carbitol). Als niedere Alkanole eignen sich mit Wasser mischbare Alkylalkohole mit 1 - 4, vorzugsweise 1 - 3, Kohlenstoffatomen, insbesondere Ethanol oder Isopropanol. Als niedere aliphatische Ketone eignen sich mit Wasser mischbare Ketone mit 3 - 5 Kohlenstoffatomen, insbesondere Aceton. Als besonders günstig erweisen sich Elutionsflüssigkeiten, in denen das organische Lösemittel Ethanol ist. In der Elutionsflüssigkeit kann ein Volumenverhältnis von mit Wasser mischbarem organischem Lösemittel zu Wasser im Bereich von 40 : 60 bis 20 : 80, vorzugsweise ca. 30 : 70 vorliegen. Die eingesetzte Menge an Elutionsmittel kann ca. 3 bis 5 Bettvolumen pro Bettvolumen hydrophobisiertes Kieselgel betragen. Zweckmäßigerweise wird die Elutionsflüssigkeit durch einen das mit dem Oestrogengemisch beladene hydrophobisierte Kieselgel enthaltenden Reaktor mit einer solchen Durchlaufgeschwindigkeit geleitet, daß die Kontaktzeit zur vollständigen Elution des Gemisches konjugierter Oestrogene ausreicht. Bei Verwendung eines Gemisches Tetrahydrofuran und/oder Ethanol mit Wasser im Vol.-Verhältnis 30 : 70 eignen sich beispielsweise Durchlaufgeschwindigkeiten von 5 bis 20 Vol.-Teilen Elutionsflüssigkeit pro 1 Vol.-Teil Kieselgel pro Stunde. Zweckmäßig wird die Durchlaufgeschwindigkeit durch Arbeiten bei leicht erhöhtem Druck, z. B. bei einem Überdruck von bis zu 0,2 bar, reguliert und das Eluat in mehreren Fraktionen aufgefangen. Die Gehalte der einzelnen Eluatfraktionen an konjugierten Oestrogenen und phenolischen Harninhaltsstoffen wie Kresolen und HPMF können auf an sich bekannte Weise durch Hochleistungsflüssigkeits-Chromatographie (= high performance liquid chromatography, abgekürzt als "HPLC") bestimmt werden.

Bei der Elution wird zunächst eine schwachgefärbte bis farblose, praktisch oestrogenfreie Vorlauffraktion erhalten, deren Menge im allgemeinen ca. einem Bettvolumen entspricht. Die Hauptmenge der konjugierten Oestrogene, beispielsweise zwischen 80 und 99 % der in dem Ausgangs-PMU vorliegenden konjugierten Oestrogene, befindet sich in den nachfolgenden dunkelgelb-braun gefärbten Haupteluatfraktionen, deren Menge im allgemeinen 1 bis 2 Bettvolumen umfaßt. In den nachfolgenden Nachlauffraktionen sind im allgemeinen nur noch Spuren an konjugierten Oestrogenen enthalten. Sofern Nachfolgefraktionen erhalten werden, welche noch einem Gehalt an konjugierten Oestrogenen von über 10 Gew.-% bezogen auf Trockensubstanz und weniger als 0,6 Gew.-% bezogen auf Trockensubstanz an Kresolen und HPMF enthalten, können diese mit dem oestrogenreichen Haupteluat zur Weiterverarbeitung vereinigt werden.

Das auf die vorstehend beschriebene Weise von dem Kieselgel abgetrennte Haupteluat enthält das im PMU auftretende natürliche Gemisch konjugierter Oestrogene neben nur einem geringen Anteil des ursprünglich in dem PMU vorhandenen Gehaltes an phenolischen Harninhaltsstoffen. Dieses Eluat kann als Ausgangsmaterial für die Herstellung von das natürliche Gemisch konjugierter Oestrogene enthaltenden Arzneimitteln dienen. Gewünschtenfalls kann das Eluat auf an sich bekannte Weise weiter eingeengt werden, um ein weitgehend von organischem Lösemittel befreites, zur galenischen Weiterverarbeitung geeignetes Konzentrat zu erhalten. Gewünschtenfalls kann auch durch Sprühtrocknung ein elutionsmittelfreies Feststoffgemisch hergestellt werden. Sofern das natürliche Gemisch konjugierter Oestrogene für die Herstellung fester Arzneimittel verwendet werden soll, kann es zweckmäßig sein, dem die konjugierten Oestrogene enthaltenden Eluat einen festen Trägerstoff bereits vor einer Konzentrierung oder Sprühtrocknung zuzumischen, um auf diese Weise ein die konjugierten Oestrogene und Trägerstoffe enthaltendes Feststoffgemisch zu erhalten. Sowohl das das Oestrogengemisch enthaltende Eluat als auch ein daraus hergestelltes Konzentrat oder sprühgetrocknetes Feststoffprodukt können in an sich bekannter Weise in feste oder flüssige galenische Zubereitungen wie beispielsweise Tabletten, Dragees, Kapseln oder Emulsionen eingearbeitet werden. Diese galenischen Zubereitungen können nach an sich bekannten Methoden hergestellt werden unter Verwendung üblicher fester oder flüssiger Trägerstoffe wie z. B. Stärke, Cellulose, Milchzucker oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln. So kann das die konjugierten Oestrogene enthaltende Produkt mit den pharmazeutischen Träger- und Hilfsstoffen auf an sich bekannte Weise vermischt werden und das Gemisch in eine geeignete Dosierungsform überführt werden.

Die nachfolgenden Beispiele sollen die Erfindung näher erläutern, ohne jedoch ihren Umfang zu beschränken.

### Beispiele 1 - 5

Allgemeine Arbeitsvorschrift zur Gewinnung eines an phenolischen Harninhaltsstoffen weitgehend abgereicherten das natürliche Gemisch der im PMU enthaltenen konjugierten Oestrogene enthaltenden Extraktes aus PMU.
**A) Vorbehandlung des PMU.**
   10 1 eines durch ein 5 cm hohes Sandbett oder durch eine Microfiltrationsanlage filtrierten PMU (Trockensubstanzgehalt (=TS) und auch mittels HPLC bestimmte Gehalte an Oestronsulfatsalz, Kresol und HPMF siehe nachfolgende Beispielstabelle) werden unter kräftigem Rühren durch Zugabe einer 50 %igen wäßrigen Natriumhydroxidlösung (Dichte bei 20° C = 1,52 g/ml) auf pH 13 eingestellt (zugegebene Menge NaOH-Lösung siehe Beispielstabelle) und eine Stunde bei Raumtemperatur gerührt. Anschließend wird die alkalische Harnflüssigkeit durch Zugabe einer konzentrierten wäßrigen Salzsäurelösung (ca. 35 %ig) auf einen pH-Wert von 8 bis 8,5 eingestellt.
**B) Adsorption des Oestrogengehaltes des PMU an RP-Kieselgel.**
   Es wird eine Säule von 50 cm Höhe mit einem Durchmesser von 5 cm mit 100 g (^ einem Volumen von ca. 193 ml = 1 Bettvolumen) RP-Kieselgel (= "Kieselgel 60/Dimethylsilanderivat" der Fa. Merck, Bestell-Nr. 7719, Korngröße 63 bis 200 µm) gefüllt. Die Säule wird zunächst mit 0,5 l Wasser, danach mit 0,25 l Methanol und nochmals mit 0,5 l Wasser gewaschen.
   Die unter A) vorbehandelte Harnflüssigkeit wird mit einem Überdruck von 0,2 bar und mit einer Durchflußgeschwindigkeit von 60 ml/Minute durch die Säule geleitet. Der Oestrogengehalt des PMU ist vollständig auf der so beladenen RP-Kieselgelsäule adsorbiert. Die ablaufende Harnflüssigkeit wird mittels HPLC auf ihren Gehalt an Oestronsulfatsalz untersucht und erweist sich als praktisch Oestrogen-frei. Die Ablauf wird verworfen.
**C) Waschen der beladenen RP-Kieselgelsäule.**
   Die beladene RP-Kieselgelsäule wird mit 2 l (^ ca. 10 Bettvolumen) einer 0,15-molaren Essigsäure/Natriumacetat-Pufferlösung gewaschen. Die Pufferlösung wird hergestellt, indem zu einer 0,15 molaren wäßrigen Essigsäurelösung festes Natriumacetat (ca. 107 g) bis zum Erreichen von pH 5 zugefügt wird. Die ablaufende Waschflüssigkeit wird mittels HPLC auf ihren Gehalt an Oestronsulfatsalz, Kresol und HPMF untersucht und enthält unter 5 % des Oestrogengehaltes des Ausgangs-PMU.
**D) Desorption der konjugierten Oestrogene von der gewaschenen RP-Kieselgelsäule und Abtrennung einer oestrogenreichen Eluatfraktion.**
   1 l der Elutionsflüssigkeit (Wasser/Lösemittel-Gemisch, Zusammensetzung siehe nachfolgende Beispielstabelle) werden mit einem Überdruck von 0,2 bar und einer Fließgeschwindigkeit von ca. 60 ml/min. durch die Säule geleitet. Das ablaufende Eluat wird in mehreren Fraktionen (Volumen je ca. 200 ml ^ ca. 1 Bettvolumen) aufgefangen. Die einzelnen Fraktionen werden mittels HPLC auf ihren Gehalt an Oestronsulfatsalz, Kresol und HPMF untersucht. Die erste Fraktion wird gesammelt, solange das Eluat farblos bis leicht gelb gefärbt erscheint. Das Volumen dieser Fraktion beträgt 150 bis 200 ml. Diese Fraktion enthält nur Spuren an Oestrogensulfatsalz.
   Sobald ein Farbwechsel des Eluats zu einer intensiven dunkelgelben bis braunen Farbe eintritt, wird die nächste Fraktion von ca. 200 ml gesammelt. In dieser Fraktion sind ca. 80 bis 98 % der gesamten auf die Säule geladenen Menge konjugierter Oestrogene enthalten. Die restlichen Fraktionen enthalten nur noch geringe Mengen an Oestrogensulfatsalz. Sie können gewünschtenfalls nach dem Abdestillieren des Lösemittelgehaltes nochmals dem Verfahrensschritt B) zugeführt werden.
   Für die die Hauptmenge der konjugierten Oestrogene enthaltende 2. Fraktion sind in der nachfolgenden Beispielstabelle jeweils der TS-Gehalt in Gew.-% und die durch HPLC bestimmten Gehalte an Oestronsulfatsalz, Kresol und HPMF angegeben. Diese Fraktion stellt einen für eine galenische Weiterverarbeitung geeigneten Extrakt dar.
**E) Regenerierung der RP-Kieselgelsäule**
   Zur Regenerierung wird die Säule zunächst mit 2 l Wasser, danach mit 0,5 l Methanol und nochmals mit 2 l Wasser gewaschen. Danach kann die Säule erneut für das Verfahren eingesetzt werden. Die Säule kann mehrmals, z. B. bis zu zehnmal, regeneriert und wieder verwendet werden.

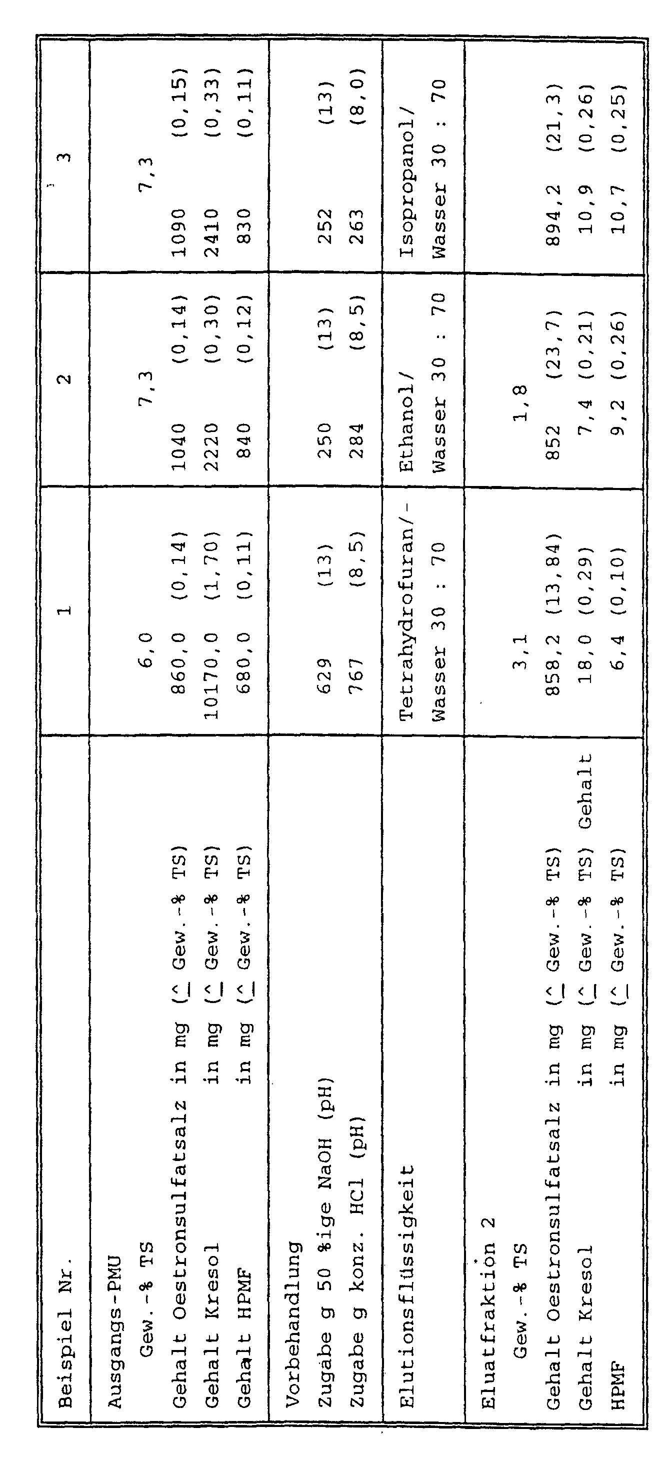

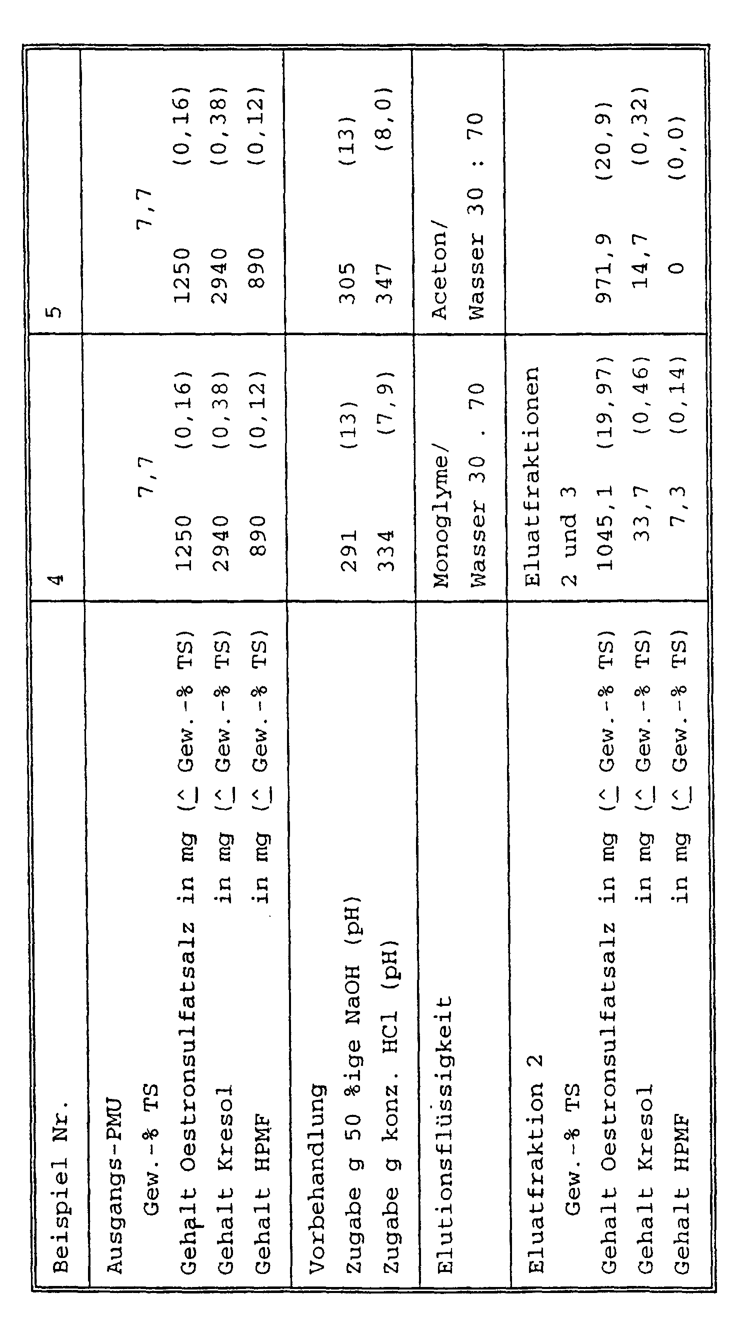

## Patentansprüche

1. Verfahren zur Gewinnung eines an phenolischen Harninhaltsstoffen abgereicherten natürlichen Gemisches konjugierter Oestrogene aus dem Harn trächtiger Stuten, **dadurch gekennzeichnet, daß** man
a) eine Harnflüssigkeit, welche den von Schleim- und Feststoffen befreiten Harn, ein eingeengtes Konzentrat dieses Harns oder ein durch Membranfiltration dieses Harns erhaltenes eingeengtes Harnretentat darstellt, mit einer zur Einstellung eines pH-Wertes von mindestens 12 ausreichenden Menge einer wasserlöslichen Base behandelt und anschließend den pH-Wert der mit der Base behandelten Harnflüssigkeit durch Zugabe einer ausreichenden Menge einer wäßrigen Säurelösung auf einen pH-Wert im Bereich von 5 bis 8,5 einstellt,
b) die mit Base vorbehandelte, auf pH 5 bis 8,5 eingestellte Harnflüssigkeit mit einer zur Adsorption des in der Harnflüssigkeit enthaltenen Gemisches konjugierter Oestrogene ausreichenden Menge eines hydrophobisierten Kieselgels kontaktiert und ein mit dem Gemisch konjugierter Oestrogene beladenes hydrophobisiertes Kieselgel von der übrigen Harnflüssigkeit abtrennt,
c) das mit dem Gemisch konjugierter Oestrogene beladene hydrophobisierte Kieselgel mit einer wässrigen auf einen pH-Bereich von 5 bis 7 eingestellten Pufferlösung wäscht, und
d) das gewaschene hydrophobisierte Kieselgel mit einer zur Desorption des daran adsorbierten Gemisches konjugierter Oestrogene ausreichenden Menge einer Elutionsflüssigkeit, welche ein Gemisch aus Wasser und einem mit Wasser mischbaren organischen Lösemittel aus der Gruppe mit Wasser mischbarer Ether, niederer Alkanole und niederer aliphatischer Ketone darstellt, kontaktiert und ein das natürliche Gemisch konjugierter Oestrogene enthaltendes Eluat von dem hydrophobisierten Kieselgel abtrennt und gegebenenfalls einengt.

2. Verfahren nach Anspruch 1, **dadurch gekennzeichnet, daß** in dem Verfahrensschritt a) die Harnflüssigkeit mit der Base, vorzugsweise mit einer wäßrigen Lösung der Base, vermischt wird und während einer zur Spaltung von in Harninhaltsstoffen enthaltenen Lactongruppierungen ausreichenden Zeitdauer auf dem pH-Wert von mindestens 12 gehalten wird.

3. Verfahren nach einem der vorstehenden Ansprüche, **dadurch gekennzeichnet, daß** als hydrophobisiertes Kieselgel ein Dimethylhydroxysilyloxyreste tragendes silanisiertes Kieselgel eingesetzt wird.

4. Verfahren nach einem der vorstehenden Ansprüche, **dadurch gekennzeichnet, daß** in dem Verfahrensschritt b) 1 Volumenteil an hydrophobisiertem Kieselgel mit einer 30 bis 80, vorzugsweise 40 bis 50, Volumenteilen Harn entsprechenden Menge Harnflüssigkeit beladen wird.

5. Verfahren nach einem der vorstehenden Ansprüche, **dadurch gekennzeichnet, daß** in dem Verfahrensschritt b) die Harnflüssigkeit mit einer Durchflußgeschwindigkeit, welche einem Durchlauf von 5 bis 20 Vol.Teilen Harn/1 Vol.Teil hydrophobisiertes Kieselgel/Stunde entspricht, durch einen das hydrophobisierte Kieselgel enthaltenden Reaktor geleitet wird.

6. Verfahren nach einem der vorstehenden Ansprüche, **dadurch gekennzeichnet, daß** die im Verfahrensschritt c) als Waschflüssigkeit eingesetzte wässrige Pufferlösung eine auf ca. pH 5 eingestellte wäßrige Essigsäure/Acetat-Pufferlösung ist.

7. Verfahren nach einem der vorstehenden Ansprüche, **dadurch gekennzeichnet, daß** im Verfahrensschritt d) als Elutionsflüssigkeit ein Gemisch aus Wasser und einem mit Wasser mischbaren organischen Lösemittel mit einem Volumenverhältnis von organischem Lösemittel zu Wasser im Bereich von 20 : 80 bis 40 : 60, insbesondere 30 : 70 eingesetzt wird.

## Claims

1. A method for obtaining a natural mixture, depleted in phenolic urine contents, of conjugated oestrogens from the urine of pregnant mares, **characterised in that**
a) a urine, which represents the urine freed of mucilaginous substances and solids, a reduced concentrate of this urine or a reduced urine retentate obtained by membrane filtration of this urine, is treated with a quantity of a water-soluble base sufficient for setting a pH value of at least 12 and then the pH value of the urine treated with the base is set to a pH value in the range of 5 to 8.5 by the addition of a sufficient quantity of an aqueous acid solution,
b) the urine which was pre-treated with base and set to pH 5 to 8.5 is contacted with a quantity of a hydrophobised silica gel sufficient for the adsorption of the mixture of conjugated oestrogens contained in the urine, and a hydrophobised silica gel laden with the mixture of conjugated oestrogens is separated off from the rest of the urine,
c) the hydrophobised silica gel laden with the mixture of conjugated oestrogens is washed with an aqueous buffer solution set to a pH range of 5 to 7, and
d) the washed hydrophobised silica gel is contacted with a quantity of an elution liquid sufficient for desorption of the mixture of conjugated oestrogens adsorbed thereon, which liquid represents a mixture of water and a water-miscible organic solvent from the group of water-miscible ethers, lower alkanols and lower aliphatic ketones, and an eluate containing the natural mixture of conjugated oestrogens is separated off from the hydrophobised silica gel and optionally reduced.

2. A method according to Claim 1, **characterised in that** in method step a) the urine is mixed with the base, preferably with an aqueous solution of the base, and is kept at the pH value of at least 12 for a period sufficient for cleavage of lactone groups contained in urine contents.

3. A method according to one of the preceding claims, **characterised in that** a silanised silica gel bearing dimethylhydroxysilyloxy radicals is used as the hydrophobised silica gel.

4. A method according to one of the preceding claims, **characterised in that** in method step b) 1 part by volume hydrophobised silica gel is laden with a quantity of urine liquid corresponding to 30 to 80, preferably 40 to 50, parts by volume urine.

5. A method according to one of the preceding claims, **characterised in that** in method step b) the urine is passed through a reactor containing the hydrophobised silica gel at a flow rate which corresponds to a throughput of 5 to 20 parts by volume urine/1 part by volume hydrophobised silica gel/hour.

6. A method according to one of the preceding claims, **characterised in that** the aqueous buffer solution used as washing liquid in method step c) is an aqueous acetic acid/acetate buffer solution set to approximately pH 5.

7. A method according to one of the preceding claims, **characterised in that** in method step d) a mixture of water and a water-miscible organic solvent having a volume ratio of organic solvent to water in the range of 20 : 80 to 40 : 60, in particular 30 : 70, is used as elution liquid.

## Revendications

1. Procédé d'obtention d'un mélange naturel d'oestrogènes conjugués, appauvri en constituants d'urine phénoliques, à partir d'urine de jument gravide, **caractérisé en ce que**
a) on traite un liquide urinaire, qui consiste en l'urine exempte des mucosités et substances solides, un concentré de cette urine ou un rétentat urinaire concentré obtenu par filtration membranaire de cette urine, avec une quantité d'une base hydrosoluble suffisante pour ajuster une valeur de pH au moins égale à 12, puis on ajuste la valeur de pH du liquide urinaire traité avec la base, par ajout d'une quantité suffisante d'une solution acide aqueuse, pour obtenir une valeur de pH dans la gamme de 5 à 8,5,
b) on met en contact le liquide urinaire pré-traité avec la base et ajusté à pH 5 à 8,5 avec une quantité d'un gel de silice rendu hydrophobe, suffisante pour adsorber le mélange d'oestrogènes conjugués contenu dans le liquide urinaire, et on sépare un gel de silice, rendu hydrophobe et chargé du mélange d'oestrogènes conjugués, du reste du liquide urinaire,
c) on lave le gel de silice rendu hydrophobe et chargé du mélange d'oestrogènes conjugués avec une solution tampon aqueuse ajustée à un pH dans la gamme de 5 à 7, et
d) on met en contact le gel de silice, rendu hydrophobe et lavé, avec une quantité d'un liquide d'élution suffisante pour la désorption du mélange d'oestrogènes conjugués qui y est adsorbé, le liquide d'élution étant un mélange d'eau et d'un solvant organique miscible à l'eau appartenant au groupe des éthers, des alcanols inférieurs et des cétones aliphatiques inférieures miscibles à l'eau, et on sépare du gel de silice rendu hydrophobe, un éluat contenant le mélange naturel d'oestrogènes conjugués, et on concentre éventuellement.

2. Procédé selon la revendication 1, **caractérisé en ce que**, dans l'étape de procédé a), le liquide urinaire est mélangé avec la base, de préférence avec une solution aqueuse de la base, et maintenu à la valeur de pH au moins égale à 12 pendant une durée suffisante pour la séparation des groupements lactone contenus dans les constituants d'urine.

3. Procédé selon l'une des revendications précédentes, **caractérisé en ce que** l'on utilise, en tant que gel de silice rendu hydrophobe, un gel de silice silané portant des radicaux diméthylhydroxysilyloxy.

4. Procédé selon l'une des revendications précédentes, **caractérisé en ce que**, dans l'étape de procédé b), on charge 1 partie en volume de gel de silice rendu hydrophobe avec une quantité de liquide urinaire correspondant à 30 à 80, de préférence à 40 à 50, parties en volume d'urine.

5. Procédé selon l'une des revendications précédentes, **caractérisé en ce que**, dans l'étape de procédé b), le liquide urinaire est dirigé à travers un réacteur, contenant le gel de silice rendu hydrophobe, à une vitesse de passage qui correspond à un débit de 5 à 20 parties en volume d'urine/1 partie en volume de gel de silice rendu hydrophobe/heure.

6. Procédé selon l'une des revendications précédentes, **caractérisé en ce que** la solution tampon aqueuse, utilisée dans l'étape de procédé c) en tant que liquide de lavage, est une solution tampon aqueuse d'acide acétique/acétate ajustée à environ pH 5.

7. Procédé selon l'une des revendications précédentes, **caractérisé en ce que**, dans l'étape de procédé d), on utilise en tant que liquide d'élution un mélange d'eau et d'un solvant organique miscible à l'eau à un rapport en volume entre solvant organique et eau dans la gamme de 20:80 à 40:60, en particulier de 30:70.
